# EUROPEAN PATENT SPECIFICATION

(11) **EP 1 214 937 B1**
(45) Date of publication and mention of the grant of the patent: **30.05.2007**
(21) Application number: 02006356.6
(22) Date of filing: 03.08.1993
(51) Int. Cl.: A61K 31/445, A61P 17/00

(54) **Terfenadine carboxylate and the treatment of dermal irritation**
Terfenadin-Carboxylat und die Behandlung von Hautreizung
Le carboxylate de la terfénadine et le traitement de l'irritation de la peau

(30) Priority: 03.08.1992 US 924156; 03.08.1992 US 924182
(43) Date of publication of application: 19.06.2002
(62) Divisional of application: 97104837.6
(73) Proprietor: Sepracor Inc., Marlborough, Massachusetts 01752 (US)
(72) Inventor: Young, James W., Palo Alto, CA 94304 (US); Gray, Nancy M., Marlborough, MA 01752 (US); Woosley, Raymond L., Washington, DC 20016 (US); Chen, Yiwang, Silver Spring, MD 20906 (US)
(74) Representative: Elend, Almut Susanne

(56) References cited:
- EP-A- 0 636 364
- EP-A- 0 636 365
- WO-A-93/21156
- WO-A-93/23047
- WO-A-95/00480
- WO-A-95/00482
- WO-A-95/10278
- FR-A- 2 453 854
- K.Y.CHAN ET AL.: "LACK OF INTERCONVERSION OF THE ENANTIOMERS OF TERFENADINE IN VIVO..." PHARM.RES., vol. 7, no. SUPP, September 1990 (1990-09), page S222 XP002046560
- J.E.COUTANT ET AL.: "DETERMINATION OF TERFENADINE AND TERFENADINE ACID METABOLITE..." J.CHROMATOGRAPHY, vol. 570, no. 1, 18 September 1991 (1991-09-18), pages 139-148, XP002046561
- K.Y.CHAN ET AL.: "DIRECT ENANTIOMERIC SEPARATION..." J.CHROMATOGRAPHY, vol. 571, no. 1-2, 1991, pages 291-297, XP002046562
- K.ZAMANI ET AL.: "ENANTIOMERIC ANALYSIS OF TERFENADINE IN RAT PLASMA BY HPLC" CHIRALITY, vol. 3, no. 6, 1991, pages 467-470, XP002046563
- K.TASAKA ET AL.: "ANTIALLERGIC EFFECTS OF TERFENADINE..." YAKURI TO CHIRYO, vol. 16, no. 6, 1988, pages 2465-2480, XP002046564
- D.A.GARTEIZ ET AL.: "PHARMACOKINETICS AND BIOTRANSFORMATION STUDIES OF TERFENADINE IN MAN" ARZNEIM.-FORSCH./DRUG RES., vol. 32(II), no. 9A, 1982, pages 1185-1190, XP000887296
- R.L.WOOSLEY: "MECHANISM OF THE CARDIOTOXIC ACTIONS OF TERFENADINE" JAMA, vol. 269, no. 12, 24 March 1993 (1993-03-24), pages 1532-1536, XP002046566 & JAMA, vol. 269, no. 12, 24 March 1993 (1993-03-24), pages 1550-1552, 269
- T.-M.CHEN ET AL.: "DETERMINATION OF THE METABOLITES OF TERFENADINE..." J.PHARM.BIOMED.ANAL., vol. 9, 1991, pages 929-933, XP002046567
- P.K.HONIG ET AL.: "CHANGES IN THE PHARMACOKINETICS..." CLIN.PHARMACOL.THER., vol. 52, no. 3, September 1992 (1992-09), pages 231-238, XP002046568
- K.TASAKA ET AL.: "INTRACELLULAR CALCIUM..." ANN.ALLERGY, vol. 56, no. 6, June 1986 (1986-06), pages 464-469, XP002046569
- M.AKAGI ET AL.: "ANTIALLERGIC EFFECTS OF TERFENADINE..." IMMUNOPHARMACOL.IMMUNOTOXICOL., vol. 9, no. 2-3, 1987, pages 259-279, XP002046570
- E.J.ARIENS: "RACEMISCHE THERAPEUTICA PROBLEEMMIDDELEN" PHARMACEUTISCH WEEKBLAD, vol. 125, no. 2, 1 June 1990 (1990-06-01), pages 552-554, XP000653502
- B.TESTA ET AL.: "RACEMATES VERSUS ENANTIOMERS IN DRUG DEVELOPMENT: DOGMATISM OR PRAGMATISM?" CHIRALITY, vol. 2, 1990, pages 129-133, XP000653443
- E.J.ARIENS: "RACEMIC THERAPEUTICS - ETHICAL AND REGULATORY ASPECTS" EUR.J.CLIN.PHARMACOL., vol. 41, no. 2, 1991, pages 89-93, XP000653474
- E.J.ARIENS: "STEREOSELECTIVITY IN PHARMACODYNAMICS AND PHARMACOKINETICS" SCHWEIZ.MED.WOCHENSCHR., vol. 120, no. 5, 3 February 1990 (1990-02-03), pages 131-134, XP000653486
- T.Y.K.CHAN: "Terfenadine-Induced Torsade de Pointes: Risk Factors and Mechanisms" JOURNAL OF PHARMACY TECHNOLOGY, vol. 13, no. 3, May 1997 (1997-05) - June 1997 (1997-06), pages 127-132, XP008007089
- N. HJORTH: "Terfenadine in the Treatment of Chronic diopathic Urticaria and Atopic Dermatitis" CUTIS, vol. 42, no. 4A, 27 October 1988 (1988-10-27), pages 29-30, XP008007086
- J.-P.RIHOUX ET AL.: "The Skin as a Target Organ for the Investigation of Antiallergic Drugs: Comparison between Cetirizine and Terfenadine" DERMATOLOGY, vol. 184, no. 2, 1992, pages 111-115, XP008007079
- D. MCTAVISH ET AL.: "Terfenadine, An Updated Review of its Pharmacological Properties and Therapeutic Efficacy" DRUGS, vol. 39, no. 4, April 1990 (1990-04), pages 552-574, XP008007081
- L.B.KRAUSE ET AL.: "The effect of terfenadine on dermographic wealing" BRITISH JOURNAL OF DERMATOLOGY, vol. 110, no. 1, January 1984 (1984-01), pages 73-79, XP008007088
- C.RIGONI ET AL.: "Trattamento delle dermatosi allergiche istamino-dipendenti con terfenadina compresse 120 mg in monosomministrazione giornaliera" GIORNALE ITALIANO DI DERMATOLOGIA E VENEREOLOGIA, vol. 124, no. 9, September 1989 (1989-09), pages XXXIX-XLI, XP008007085
- A.SABBAH: "Renaissance des anti-H1" ALLERGIE ET IMMUNOLOGIE, vol. 24, no. 6, June 1992 (1992-06), pages 224-230, XP008007080
- J.BOYLE ET AL.: "Acrivastine versus Terfenadine in the Treatment of Symptomatic Dermographism - a Double-blind, Placebo-controlled Study" THE JOURNAL OF INTERNATIONAL MEDICAL RESEARCH, vol. 17, no. suppl. 2, 1989, pages 9B-13B, XP008007087
- D.RAMPE ET AL.: "Effects of Terfenadine and Its Metabolites on a Delayed Rectifier K+ Channel Cloned from Human Heart" MOL.PHARMACOL., vol. 44, no. 6, 1993, pages 1240-1245, XP008007138

## Description

This invention relates to novel pharmaceutical compositions containing 4-[1-hydroxy-4-(4-hydroxydiphenylmethyl-1-piperidinyl)butyl]-α,α-dimethylbenzeneacetic acid and its optically pure derivatives. These compositions possess potent antihistaminic activity and are useful in treating allergic rhinitis, asthma and other allergic disorders while avoiding adverse effects associated with the administration of other α-aryl-4-substituted piperidinoalkanol derivatives, such as terfenadine, including but not limited to cardiac arrhythmias, drowsiness, nausea, fatigue, weakness and headache. Also, these compositions, in combination with non-steroidal anti-inflammatory agents or other non-narcotic analgesics, are useful for the treatment of cough, cold, cold-like, and/or flu symptoms and the discomfort, headache, pain, fever, and general malaise associated therewith. The aforementioned combinations may optionally include one or more other active components including a decongestant, cough suppressant/antitussive, or expectorant.

Additionally, these novel pharmaceutical compositions containing 4-[1-hydroxy-4-(4-hydroxydiphenylmethyl-1-piperidinyl)butyl]-α,α-dimethylbenzeneacetic acid and its optically pure derivatives are useful in treating motion sickness, vertigo, diabetic retinopathy, small vessel complications due to diabetes and such other conditions as may be related to the activity of these derivatives as antagonists of the H-1 histamine receptor while avoiding the adverse effects associated with the administration of other α-aryl-4-substituted piperidinoalkanol derivatives, such as terfenadine.

Also disclosed are methods for treating the above-described conditions in a human while avoiding the adverse effects that are associated with the administration of other α-aryl-4-substituted piperidinoalkanol derivatives, such as terfenadine, by administering the aforementioned pharmaceutical compositions containing 4-[1-hydroxy-4-(4-hydroxydiphenylmethyl-1-piperidinyl)butyl]-α,α-dimethylbenzeneacetic acid or its optically pure isomers to said human.

The active compounds of these compositions and methods are metabolic derivatives of terfenadine. Chemically, these derivatives are 4-[1-hydroxy-4-(4-hydroxydiphenylmethyl-1-piperidinyl)butyl]-α,α-dimethylbenzeneacetic acid and the optical isomers of this compound. These compounds are described in Garteiz et al., Arzneimittel-Forschung/Drug Research, 32: 1185-1190 (1982). Chemically, the optical isomers of the compound are R-(+)-4-[1-hydroxy-4-(4-hydroxydiphenylmethyl-1-piperidinyl)butyl]-α,α-dimethylbenzeneacetic acid and S-(-)-4-[1-hydroxy-4-(4-hydroxydiphenylmethyl-1-piperidinyl)butyl]-α,α-dimethylbenzeneacetic acid.

### Steric Relationship and Drug Action

Many organic compounds exist in optically active form, i.e., they have the ability to rotate the plane of plane-polarized light. In describing an optically active compound, the prefixes D and L or R and S are used to denote the absolute configuration of the molecule about its chiral center(s). The prefixes d and l or (+) and (-) are employed to designate the sign of rotation of plane-polarized light by the compound, with (-) or l meaning that the compound is levorotatory. A compound prefixed with (+) or d is dextrorotatory. For a given chemical structure, these compounds, called stereoisomers, are identical except that they are mirror images of one another. A specific stereoisomer may also be referred to as an enantiomer, and a mixture of such isomers is often called an enantiomeric or racemic mixture.

Stereochemical purity can be of importance in the field of pharmaceuticals, where 12 of the 20 most prescribed drugs exhibit chirality. A case in point is provided by the L-form of the β-adrenergic blocking agent, propranolol, which is known to be 100 times more potent than the D-enantiomer.

Furthermore, optical purity can be important since certain isomers may actually be deleterious rather than simply inert. For example, it has been suggested that the D-enantiomer of thalidomide was a safe and effective sedative when prescribed for the control of morning sickness during pregnancy, while the corresponding L-enantiomer has been believed to be a potent teratogen.

The enantiomers of 4-[1-hydroxy-4-(4-hydroxydiphenylmethyl-1-piperidinyl)butyl]-α,α-dimethylbenzeneacetic acid are disclosed in Zamani et al., Chirality 3: 467-470 (1991). This reference states that the (R)-enantiomer of an orally administered racemic terfenadine was preferentially oxidized in rats to form a carboxylic acid metabolite enriched in the (R)-enantiomer. The enantiomers of 4- [1-hydroxy-4-(4-hydroxydiphenylmethyl-1-piperidinyl)butyl]-α,α-dimethylbenzeneacetic acid are also disclosed in Chan et al., J. Chromatog. 571: 291-297 (1991). This reference states that terfenadine does not undergo any stereoselective isomeric interconversion in man.

Terfenadine is an antagonist of the H-1 histamine receptor protein. Histamine receptor proteins occur in two well-identified forms in tissues as H-1 and H-2 receptors. The H-1 receptors are those that mediate the response antagonized by conventional antihistamines. H-1 receptors are present in the guinea pig ileum, the skin of Rhesus monkeys, and the bronchial smooth muscle of guinea pig. Terfenadine antagonizes the effect of histamine in the guinea pig isolated ileum, suppresses histamine-induced whealing in the skin of Rhesus monkeys, and protects against histamine induced lethality in the guinea pig.

Through H-2 receptor-mediated responses, histamine stimulates gastric acid secretion in the guinea pig and the chronotropic effect in isolated guinea pig atria. Terfenadine has no effect on histamine-induced gastric acid secretion, nor does it alter the chronotropic effect of histamine on atria. Thus, terfenadine has no apparent effect on the H-2 histamine receptor. See Cheng et al., Drug Development Research, 2: 181-196 (1982).

Terfenadine is well absorbed but is extensively metabolized. See Okerholm et al., Biopharmaceutics and Drug Distribution, 2: 185-190 (1981). Two main metabolites have been identified and it has been suggested that one of the metabolites, 4-[1-hydroxy-4-[4-(hydroxydiphenylmethyl-1-piperidinyl)butyl]-α,α-dimethylbenzeneacetic acid, may show antihistaminic activity, in vitro, but no actual data have been published. See Garteiz et al., Arzneimittel-Forschung/Drug Research, 32: 1185-1190 (1982).

On the basis of its antihistaminic activity, researchers evaluated the effect of terfenadine in the treatment of allergic rhinitis. Clinical trials of efficacy indicated that terfenadine is slightly less effective than chlorpheniramine, another H-1 antagonist. See Connell, Pharmacotherapy, 5: 201-208 (1985).

It has also been suggested that terfenadine would be useful for the treatment of asthma. In guinea pigs, the increase in airway resistance caused by LTD₄ (leukotriene D₄) was suppressed by terfenadine. See Akagi et al., Oyo Yakuri, 35: 361-371 (1988).

Terfenadine may also be useful for the treatment of motion sickness and vertigo. Some antihistamines have been found to be effective for the prophylaxis and treatment of motion sickness. See Wood, Drugs, 17: 471-479 (1979). Some antihistamines have also proven useful for treating vestibular disturbances, such as Meniere's disease, and in other types of vertigo. See Cohen et al., Archives of Neurology, 27: 129-135 (1972).

In addition, terfenadine may be useful in the treatment of diabetic retinopathy and other small vessel disorders associated with diabetes mellitus. In tests on rats with streptozocin-induced diabetes, treatment by antihistamines prevented the activation of retinal histamine receptors which have been implicated in the development of diabetic retinopathy. The use of antihistamines to treat retinopathy and small vessel disorders associated with diabetes mellitus is disclosed in U.S. Patent No. 5,019,591.

It has also been suggested that terfenadine, in combination with non-steroidal anti-inflammatory agents or other non-narcotic analgesics, would be useful for the treatment of cough, cold, cold-like and/or flu symptoms and the discomfort, pain, headache, fever, and general malaise associated therewith. The use of pharmaceutical compositions containing terfenadine and non-narcotic analgesics or non-steroidal anti-inflammatory agents such as aspirin, acetaminophen, and ibuprofen are described in U.S. Patent Nos. 4,783,465 and 4,829,064. These compositions for the treatment of the above-described symptoms may optionally include one or more other active components including a decongestant (such as pseudoephedrine), a cough suppressant/antitussive (such as dextromethorphan) or an expectorant (such as guaifenesin).

Many antihistamines cause somewhat similar adverse effects. These adverse effects include but are not limited to sedation, gastrointestinal distress, dry mouth, and constipation or diarrhea. Terfenadine has been found to cause relatively less sedation, gastrointestinal distress, dry mouth, and constipation or diarrhea, as compared with other antihistamines.

However, the administration of terfenadine to a human has been found to cause other adverse effects. These adverse effects include but are not limited to cardiac arrhythmias, including ventricular tachyarrhythmias, torsades de pointes, and ventricular fibrillation. Recently, clinical practitioners have noted an increase in the occurrence of these cardiac arrhythmias upon coadministration of terfenadine with other drugs such as ketoconazole and erythromycin or upon overdose of terfenadine. See Brian P. Monahan et. al. in JAMA, 5th Dec 1990, Vol. 264, No. 21, p-p 2788-90 and Sandra Knowles in the Canadian Journal of Hospital Pharmacy - Vol. 45, No. 1, 1st Feb 1992, p. 33

Thus, it would be particularly desirable to find a compound with the advantages of terfenadine which would not have the aforementioned disadvantages.

It has now been discovered that 4-[1-hydroxy-4-(4-hydroxydiphenylmethyl-1-piperidinyl)butyl]-α,α-dimethylbenzeneacetic acid, and its optically pure isomers (hereinafter referred to as "the metabolic derivative of terfenadine" and "optically pure isomers of the metabolic derivative of terfenadine") are effective antihistamines. It has also been discovered that pharmaceutical compositions containing the metabolic derivative of terfenadine or its optically pure isomers are useful in treating allergic disorders and such other conditions as may be related to the composition's activity as an antihistamine, including but not limited to allergic rhinitis, solar urticaria, and symptomatic dermographism.

Furthermore, it has now also been discovered that the metabolic derivative of terfenadine or its optically pure isomers are useful in treating asthma. Also, these compounds are useful for the treatment of motion sickness and vertigo and in treating such disorders as retinopathy and small vessel disorders associated with diabetes mellitus. The present invention also discloses methods for treating the above-described conditions in a human, while avoiding the adverse effects that are associated with terfenadine, including but not limited to cardiac arrhythmias, sedation, gastrointestinal distress, dry mouth, and constipation or diarrhea, by administering the metabolic derivative of terfenadine or its optically pure isomers to said human.

It has also been discovered that the metabolic derivative of terfenadine and its optically pure isomers, in combination with non-steroidal anti-inflammatory agents or other non-narcotic analgesics, are useful for the treatment of cough, cold, cold-like and/or flu symptoms and the discomfort, pain, headache, fever, and general malaise associated therewith. The use of pharmaceutical compositions of the invention, containing (1) the metabolic derivative of terfenadine or its optically pure isomers and (2) non-narcotic analgesics or non-steroidal anti-inflammatory agents such aspirin, acetaminophen or ibuprofen, may optionally include one or more other active components including a decongestant (such as pseudoephedrine), a cough suppressant/antitussive (such as dextromethorphan) or an expectorant (such as guaifenesin).

The present invention discloses a method of treating a human afflicted by or susceptible to an allergic disorder while avoiding the concomitant liability of adverse effects associated with the administration of terfenadine, which comprises administering to said human afflicted by or susceptible to an allergic disorder an amount of one or more compounds selected from the group of the metabolic derivative of terfenadine, optically pure isomers of the metabolic derivative of terfenadine, and pharmaceutically acceptable salts thereof, said amount being sufficient to treat said allergic disorder, but insufficient to cause the adverse effects associated with terfenadine.

Accordingly and in a first aspect, the present invention provides a pharmaceutical composition in the form of an oral solid preparation comprising 20mg to 200mg of a pharmaceutically acceptable salt of a compound of formula I: 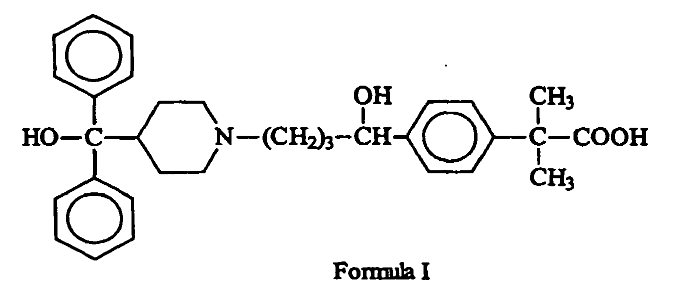 and an inorganic acid selected from the group consisting of hydrochloric acid, hydrobromic acid, hydroiodic acid, sulphuric acid and phosphoric acid, and a pharmaceutically acceptable carrier or excipient. Preferably the pharmaceutical composition is suitable for use in treating dermal irritation, while avoiding the induction of cardiac arrhythmia.

The compounds of formula I include the metabolic derivative of terfenadine and the optically pure isomers of the metabolic derivative of terfenadine as aforesaid.

Prior to the present invention, those skilled in the art would have expected compounds of formula I to have induced a form of cardiac arrhythmia known as Torsades de Pointes (as reported in Brian P. Monahan et. al., in JAMA, 5th Dec 1990, Vol. 264, No. 21, p-p 2788-90 and Sandra Knowles in The Canadian Journal of Hospital Pharmacy, Vol. 45, No. 1, 1 Feb 1992, p.33), since this potentially lethal arrhythmia was considered to be a "class effect" among non-sedating anti-histamines, in the sense that the arrhythmogenicity was considered to be coupled to the anti-histaminic potency of such compounds. Accordingly, the fact that the compositions, in accordance with the present invention, do not induce any such cardiac arrhythmias is a new, highly useful and surprising technical effect, which enables the inventive compositions to be administered to individuals susceptible to cardiac arrhythmia, and in potentially larger doses than those non-sedating anti-histamines, such as terfenadine, in common use at the present time.

The anti-histaminic treatment can be a method of treating a human afflicted by or susceptible to: an allergic disorder; motion sickness; vertigo; retinopathy, or another small vessel disease associated with diabetes mellitus; cough, cold, cold-like or flu symptoms; or the discomfort, pain, fever or general malaise associated therewith. In embodiments, the invention can relate to any one, any combination, or all of the aforementioned methods of treatment.

Preferably, the invention relates to the treatment of an allergic disorder which, preferably, is asthma or allergic rhinitis.

In preferred embodiments, the anti-histaminic treatment comprises the administration of a compound of formula I, in an amount of 1-500mg/day and, preferably, in an amount of 20-200mg/day. When the inventive composition is for use in a method of treating asthma, or retinopathy or another small vessel disease associated with diabetes mellitus, sufficient of the inventive composition should be administered such that the compound of formula I is provided to the patient in an amount of 0.01-500mg/day and, preferably, in an amount of 0.1-200mg/day.

Particularly when the anti-histaminic treatment is that of cough, cold, cold-like or flu symptoms or the discomfort, pain, fever or general malaise associated therewith, in a human, the composition can further comprise a therapeutically effective amount of a non-steroidal anti-inflammatory agent or a non-narcotic analgesic, such as acetylsalicylic acid, acetaminophen, ibuprofen, ketoprofen, or naproxen, or a pharmaceutically acceptable salt thereof. Alternatively, or additionally, a composition in accordance with the present invention can further comprise a therapeutically effective amount of a decongestant, such as pseudoephedrine, or a pharmaceutically acceptable salt thereof.

In a preferred embodiment, the inventive composition comprises from 20mg to 200mg of a compound of formula I and from 25mg to 600mg of an anti-inflammatory agent or an analgesic. When the inventive composition comprises a therapeutically effective amount of a decongestant, it, preferably, comprises from 20mg to 200mg of a compound of formula I and from 5mg to 150mg of the decongestant.

In preferred embodiments of the present invention, the compound of formula I is in the form of a single optical isomer and the inventive composition is substantially free of the other such isomer. In such an embodiment, the compound of formula I, preferably, is selected from the group consisting of: R-(+)-4-[1-hydroxy-4-(4-hydroxydiphenylmethyl-1-piperidinyl)butyl]-α,α-dimethylbenzeneacetic acid and pharmaceutically acceptable salts thereof, and the composition is substantially free of the S stereoisomer of the selected compound; or, is selected from the group consisting of S-(-)-4-[1-hydroxy-4-(4-hydroxydiphenylmethyl-1-piperidinyl)butyl]-α,α-dimethylbenzeneacetic acid and pharmaceutically acceptable salts thereof, and the composition is substantially free of the R stereoisomer of the selected compound.

Preferably, the compound of formula I comprises 90% or more of the selected R- or S-stereoisomer.

In a second aspect, the present invention provides a use of a composition comprising a compound of formula I: 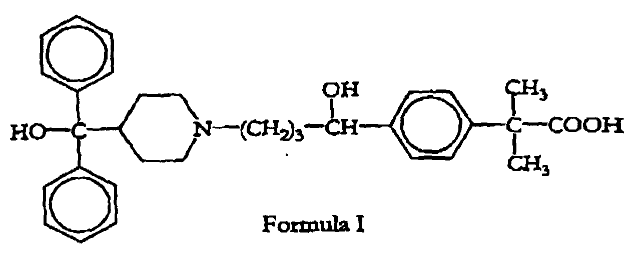 or a pharmaceutically acceptable salt thereof, for the preparation of a medicament for use in providing symptomatic relief from dermal irritation associated with an allergic disorder, cough, cold or flu, wherein the induction of cardiac arrhythmia is avoided, said treatment comprising administering a therapeutically effective amount of a compound of formula I to a human patient whose hepatic function is not impaired.

Terfenadine has antihistaminic activity and provides therapy and a reduction of symptoms for a variety of conditions and disorders related to allergic disorders, diabetes mellitus and other conditions; however, this drug, while offering the expectation of efficacy, causes adverse effects. Utilizing the metabolic derivative of terfenadine or its substantially optically pure isomers results in clearer dose-related definitions of efficacy, diminished adverse effects, and accordingly, an improved therapeutic index. It is, therefore, more desirable to use the metabolic derivative of terfenadine or an optically pure isomer thereof, than to use terfenadine itself.

The term "adverse effects" includes, but is not limited to cardiac arrhythmias, sedation, gastrointestinal distress, dry mouth, constipation, and diarrhea. The term "cardiac arrhythmias" includes, but is not limited to ventricular tachyarrhythmias, torsades de pointes, and ventricular fibrillation.

The term "substantially free of the S stereoisomer" as used herein means that the metabolic derivative of terfenadine in a composition contains at least 90% by weight of the R isomer of the metabolic derivative of terfenadine, and 10% by weight or less of the S derivative. In a preferred embodiment, the term "substantially free of the S stereoisomer" means that the metabolic derivative of terfenadine in a composition contains at least 99% by weight of the R isomer of the metabolic derivative of terfenadine, and 1% or less of the S isomer. In another preferred embodiment, the term "substantially free of the S stereoisomer" as used herein means that the metabolic derivative of terfenadine in a composition contains greater than 99% by weight of the R isomer of the metabolic derivative of terfenadine and less than 1% by weight of the S derivative. The terms "substantially optically pure R isomer of the metabolic derivative of terfenadine" and "optically pure .R isomer of the metabolic derivative of terfenadine" are also encompassed by the above-described definitions.

The term "substantially free of the R stereoisomer" as used herein means that the composition contains at least 90% by weight of the S isomer of the metabolic derivatives of terfenadine, and 10% by weight or less of the R derivative. In a preferred embodiment, the term "substantially free of the R stereoisomer" means that the composition contains at least 99% by weight of the S isomer of the metabolic derivative of terfenadine, and 1% or less of the R isomer. In another preferred embodiment, the term "substantially free of the R stereoisomer" as used herein means that the composition contains greater than 99% by weight of the S isomer of the metabolic derivative of terfenadine and less than 1% by weight of the R derivative. These percentages are based upon the total amount of metabolic derivative of terfenadine in the composition. The terms "substantially optically pure S isomer of the metabolic derivative of terfenadine" and "optically pure S isomer of the metabolic derivative of terfenadine" are also encompassed by the above-described definitions.

The phrase "therapeutically effective amount" means that amount of one or more of the compounds of the invention which provides a therapeutic benefit in an anti-histaminic treatment, including the treatment or management of allergic disorders, asthma, retinopathy or other small vessel disorders associated with diabetes mellitus, motion sickness, vertigo, or cough, cold, cold-like, and/or flu symptoms and the discomfort, pain, fever, and general malaise associated therewith. Examples of allergic disorders include, but are not limited to, allergic rhinitis, solar urticaria, and symptomatic dermographism. The symptoms associated with these allergic disorders and the cough, cold, cold-like, and/or flu symptoms include, but are not limited to, sneezing, rhinorrhea, lacrimation, and dermal irritation. The term "asthma" is defined as a disorder characterized by increased responsiveness of the trachea and bronchi to various stimuli which results in symptoms which include wheezing, cough, and dyspnea. The term "vertigo" as used herein means the dizziness associated with, but not limited to motion, height, and changes in body position. The term "diabetic retinopathy" or "retinopathy associated with diabetes mellitus" is that disorder caused by increased permeability of the capillaries in the eye which leads to hemorrhages and edema in the eye and can lead to blindness. The term "small vessel disorders associated with diabetes mellitus" includes, but is not limited to diabetic retinopathy and peripheral vascular disease.

The separation of the optically pure isomers of the metabolic derivative of terfenadine may be effected by resolution of the racemic mixture of enantiomers of the metabolic derivative of terfenadine using conventional means such as an optically active resolving acid. Furthermore, the optically pure isomers of the metabolic derivative of terfenadine can be prepared from the racemic mixture by enzymatic biocatalytic resolution. See, for example, United States Patent Nos. 5,057,427 and 5,077,217, the disclosures of which are incorporated by reference.

The magnitude of a prophylactic or therapeutic dose of the metabolic derivative of terfenadine, or an optically pure isomer thereof, in the acute or chronic management of disease will vary with the severity of the condition to be treated and the route of administration. The dose, and perhaps the dose frequency, will also vary according to the age, body weight, and response of the individual patient. In general, the total daily dose range, for the conditions described herein, is from about 0.01 mg to about 500 mg administered in single or divided doses orally, topically, transdermally, or locally by aerosol. For example, a preferred oral daily dose range should be from about 1 mg to about 500 mg, while most preferably an oral daily dose range should be between about 20 mg and about 200 mg. It is further recommended that children, patients aged over 65 years, and those with impaired renal or hepatic function initially receive low doses, and that they then be titrated based on individual response(s) or blood level(s). It may be necessary to use dosages outside these ranges in some cases as will be apparent to those skilled in the art. Further, it is noted that the clinician or treating physician will know how and when to interrupt, adjust, or terminate therapy in conjunction with individual patient response.

The various terms "an amount sufficient to alleviate said allergic disorder but insufficient to cause said adverse effects "," an amount sufficient to alleviate said asthma but insufficient to cause said adverse effects " , " an amount sufficient to alleviate said motion sickness but insufficient to cause said adverse effects "and" an amount sufficient to alleviate said retinopathy or other small vessel diseases associated with diabetes mellitus but insufficient to cause said adverse effects" are encompassed by the above-described dosage amounts and dose frequency schedule. In addition, the terms "a pharmaceutical composition for use in the treatment of cough, cold, cold-like and/or flu symptoms and the discomfort, pain, fever and general malaise associated therewith, in a human, said composition comprising (i) a therapeutically effective amount of the metabolic derivative of terfenadine, or an optically pure isomer thereof, with (ii) a therapeutically effective amount of at least one non-steroidal anti-inflammatory agent or non-narcotic analgesic," and "a pharmaceutical composition for use in the treatment of cough, cold, cold-like and/or flu symptoms and the discomfort, pain, fever and general malaise associated therewith, in a human, said composition comprising (i) a therapeutically effective amount of the metabolic derivative of terfenadine, or an optically pure isomer, with (ii) a therapeutically effective amount of a decongestant " as well as the term "therapeutically effective amount of at least one α-aryl-4-substituted piperidinoalkanol derivative" are also encompassed by the above-described dosage amounts and dose frequency schedule.

Any suitable route of administration may be employed for providing the patient with an effective dosage of the inventive composition. For example, oral, rectal, parenteral, transdermal, subcutaneous, intramuscular, and like forms of administration may be employed. Dosage forms include tablets, troches, dispersions, suspensions, solutions, capsules, patches, and the like.

The pharmaceutical compositions of the present invention comprise the metabolic derivative of terfenadine, or an optically pure isomer thereof as active ingredient, or a pharmaceutically acceptable salt thereof, and also contain a pharmaceutically acceptable carrier or excipient, and optionally other therapeutic ingredients.

The term "pharmaceutically acceptable salts" includes within its ambit salts prepared from pharmaceutically acceptable non-toxic acids or bases including inorganic acids or bases or organic acids or bases. Examples of such inorganic acids are hydrochloric, hydrobromic, hydroiodic, sulfuric, and phosphoric. Appropriate organic acids may be selected, for example, from aliphatic, aromatic, carboxylic and sulfonic classes of organic acids, examples of which are formic, acetic, propionic, succinic, glycolic, glucoronic, maleic, furoic, glutamic, benzoic, anthranilic, salicylic, phenylacetic, mandelic, embonic (pamoic), methanesulfonic, ethanesulfonic, pantothenic, benzenesulfonic, stearic, sulfanilic, algenic, and galacturonic. Examples of such inorganic bases include metallic salts made from aluminum, calcium, lithium, magnesium, potassium, sodium, and zinc. Appropriate organic bases may be selected, for example, from N,N'-dibenzylethylenediamine, chloroprocaine, choline, diethanolamine, ethylenediamine, meglumaine (N-methylglucamine), lysine and procaine.

The compositions of the present invention include compositions such as suspensions, solutions and elixirs; aerosols; or carriers such as starches, sugars, microcrystalline cellulose, diluents, granulating agents, lubricants, binders, disintegrating agents, and the like, in the case of oral solid preparations (such as powders, capsules, and tablets), with the oral solid preparations being preferred over the oral liquid preparations. The most preferred oral solid preparations are tablets.

Because of their ease of administration, tablets and capsules represent the most advantageous oral dosage unit form, in which case solid pharmaceutical carriers are employed. If desired, tablets may be coated by standard aqueous or nonaqueous techniques.

In addition to the common dosage forms set out above, the compounds of the present invention may also be administered by controlled release means and/or delivery devices such as those described in U.S. Patent Nos. 3,845,770; 3,916,899; 3,536,809; 3,598,123; and 4,008,719.

Pharmaceutical compositions of the present invention suitable for oral administration may be presented as discrete units such as capsules, cachets, or tablets, or aerosol sprays, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active or dispersing agent. Molded tablets may be made by molding, in a suitable machine, a mixture of the powdered compound moistened with an inert liquid diluent. Desirably, each tablet contains from about 10 mg to about 150 mg of the active ingredient, and each cachet or capsule contains from about 10 mg to about 150 mg of the active ingredient, i.e. the metabolic derivative of terfenadine. Most preferably, the tablet, cachet or capsule contains either one of three dosages, 30 mg, 60 mg or 90 mg of the active ingredient.

The invention is further defined by reference to the following examples describing in detail the preparation of the compound and the compositions of the present invention, as well as their utility. It will be apparent to those skilled in the art that many modifications, both to materials and methods, may be practiced which are within the scope of this invention.

### EXAMPLES

### Example 1

### A. Preparation of methyl R-4-[1-hydroxy-4-(4-hydroxydiphenylmethyl-1-piperidinyl)butyl]-α,α-dimethylbenzeneacetate.

4-(α-hydroxy-α-phenylbenzyl)piperidine (4.3 gm) was combined with methyl p-(4-chloro-1-oxobutyl)-α,α-dimethylbenzeneacetate (4.5 gm), potassium bicarbonate (2.9 gm), potassium iodide (ca. 50 mg), and methyl isobutyl ketone (50 ml) and heated to reflux for 48 hours. Additional 4-(α-hydroxy-α-phenylbenzyl)piperidine (1.1 gm) was added, and heating was continued for an additional 48 hours. Upon cooling the mixture to room temperature, water was added and the pH of the solution was adjusted to ca. 12 by addition of aqueous sodium hydroxide. The mixture was extracted with ethyl acetate. The ethyl acetate solution was washed with saturated aqueous sodium bicarbonate and brine and dried over sodium sulfate. The ethyl acetate was removed on a rotary evaporator and the residue was treated with 25% ethyl acetate in hexane. The resulting precipitate was filtered and air dried to give methyl 4-[1-oxo-4-(4-hydroxydiphenylmethyl-1-piperidinyl)butyl]-α,α-dimethylbenzeneacetate. This intermediate precipitate (2.4 gm) was combined with tetrahydrofuran (10 ml) and (+)-β-chlorodiisopinocamphenylborane (4.5 gm) and stirred for 48 hours. Methanol (10 ml) and sodium bicarbonate (1.5 gm) were added to the reaction solution, and the mixture was stirred for 12 hours. The mixture was diluted with ethyl acetate (200 ml) and washed with saturated aqueous sodium bicarbonate to give methyl R-4-[1-hydroxy-4-(4-hydroxydiphenylmethyl-1-piperidinyl)butyl]-α,α-dimethylbenzeneacetate.

### B. R-(+)-4-[1-hydroxy-4-(4-hydroxydiphenylmethyl-1-piperidinyl)butyl]-α,α-dimethylbenzeneacetic acid [R-(+)-terfenadine carboxylate].

Methyl R-4-[1-hydroxy-4-(4-hydroxydiphenylmethyl-1-piperidinyl)butyl]-α,α-dimethylbenzeneacetate (1.2 gm) was combined with potassium hydroxide (0.4 gm) and ethanol (5 ml), and the mixture was heated to reflux for 7 hours. The ethanol was removed on a rotary evaporator and the residue was dissolved in water (2 ml). The aqueous solution was acidifed with glacial acetic acid to provide a solid which was recrystallized from 1:1 methanol/ethyl acetate to give R-4-[1-hydroxy-4-(4-hydroxydiphenylmethyl-1-piperidinyl)butyl]-α,α-dimethylbenzeneacetic acid (R-terfenadine carboxylate) (mp=213-215°C).

### C. Preparation of methyl S-4-[1-hydroxy-4-(4-hydroxydiphenylmethyl-1-piperidinyl)butyl]-α,α-dimethylbenzeneacetate.

4-(α-hydroxy-α-phenylbenzyl)piperidine (4.3 gm) was combined with methyl p-(4-chloro-1-oxobutyl)-α,α-dimethylbenzene-acetate (4.5 gm), potassium bicarbonate (2.9 gm), potassium iodide (ca. 50 mg), and methyl isobutyl ketone (50 ml) and heated to reflux for 48 hours. Additional 4-(α-hydroxy-α-phenylbenzyl)piperidine (1.1 gm) was added, and heating was continued for an additional 48 hours. Upon cooling the mixture to room temperature, water was added and the pH of the solution was adjusted to ca. 12 by addition of aqueous sodium hydroxide. The mixture was extracted with ethyl acetate. The ethyl acetate solution was washed with saturated aqueous sodium bicarbonate and brine and dried over sodium sulfate. The ethyl acetate was removed on a rotary evaporator and the residue was treated with 25% ethyl acetate in hexane. The resulting precipitate was filtered and air dried to give methyl 4-[1-oxo-4-(4-hydroxydiphenylmethyl-1-piperidinyl)butyl]-α,α-dimethylbenzeneacetate. This intermediate precipitate (2.4 gm) was combined with tetrahydrofuran (10 ml) and (-)-β-chlorodiisopinocamphenylborane (4.5 gm) and stirred for 48 hours. Methanol (10 ml) and sodium bicarbonate (1.5 gm) were added to the reaction solution and the mixture was stirred for 12 hours. The mixture was diluted with ethyl acetate (200 ml) and washed with saturated aqueous sodium bicarbonate to give methyl S-4-[1-hydroxy-4-(4-hydroxydiphenylmethyl-1-piperidinyl)butyl]-α,α-dimethylbenzeneacetate. If the aforementioned intermediate precipitate were to be reacted with racemic β-chlorodiisopinocamphenylborane, then a racemic mixture of methyl 4-[1-hydroxy-4-(4-hydroxydiphenylmethyl-1-piperidinyl)butyl]-α,α-dimethylbenzeneacetate would be produced.

### D. S-(-)-4-[1-hydroxy-4-(4-hydroxydiphenylmethyl-1-piperidinyl)butyl]-α,α-dimethylbenzeneacetic acid [S-(-)-terfenadine carboxylate].

Methyl S-4-[1-hydroxy-4-(4-hydroxydiphenylmethyl-1-piperidinyl)buty]-α,α-dimethylbenzeneacetate (1.2 gm) was combined with potassium hydroxide (0.4 gm) and ethanol (5 ml) and the mixture was heated to reflux for 7 hours. The ethanol was removed on a rotary evaporator and the residue was dissolved in water (2 ml). The aqueous solution was acidifed with glacial acetic acid to provide a solid which was recrystallized from 1:1 methanol/ethyl acetate to give S-(-)-4-[1-hydroxy-4-(4-hydroxydiphenylmethyl-1-piperidinyl)butyl]-α,α-dimethylbenzeneacetic acid (S-terfenadine carboxylate) (mp=215-218°C).

### Example 2

Activities of the compounds of the invention at the histamine H₁-receptor were assessed using the [³H]pyrilamine binding assay as described in Chang et al., J. Neurochem. 32: 1653-1663 (1979). Briefly, membranes from bovine cerebellum were incubated with [³H] pyrilamine and varying concentrations of test compound. The reactions were carried out in 50 mM sodium phosphate buffer (pH 7.5) at 25° C for 30 minutes. The reaction was terminated by rapid vacuum filtration onto glass fiber filters. Radioactivity trapped on the filters was determined and compared to control values to as certain the interaction of the test compound with the H₁-receptor. Results were as follows:

| Compound | Percent Inhibition (at various concentrations) | | |
|---|---|---|---|
| | 10⁻⁹ M | 10⁻⁷ M | 10⁻⁵ M |
| R,S-terfenadine | 11.0 | 28.7 | 86.9 |
| R-(+)-terfenadine | 11.4 | 19.4 | 90.3 |
| R-(+)-terfenadine carboxylate | 12.4 | 45.2 | 87.3 |
| S-(-)-terfenadine | 3.2 | 24.4 | 92.8 |
| S-(-)-terfenadine carboxylate | 8.1 | 54.1 | 88.7 |

### Example 3

Single ventricular myocytes were obtained from isolated cat hearts by conventional techniques. The rod-shaped single cells were maintained in a HEPES buffer and they were "patch clamped" using suction pipettes. A Patch-Clamp L/M-PEC 7 amplifier was used to record current tracings and the recording electrodes were filled with a solution of potassium aspartate. Voltage clamp pulses and data acquisition were controlled by a Sperry PC/IT Computer running P Clamp software. A minimum of 4 cells were studied at each test concentration of the following drugs: racemic terfenadine, racemic terfenadine carboxylate, and quinidine (as a reference compound). Results were as follows:

| | Conc (µM) | Block of the delayed rectifier potassium current (%) |
|---|---|---|
| Terfenadine | 0.01 | 12 ± 9.3 |
| | 0.10 | 39.5 ± 9.8 |
| | 1.00 | 92.6 (92.5; 92.8) |
| | | |
| Terfenadine carboxylate | 0.01 | 0 ± 0 |
| | 0.10 | 0 ± 0 |
| | 1.00 | 0 ± 0 |

These results show that terfenadine carboxylate, surprisingly, is not liable to cause cardiac arrhythmia, at dose levels at which there is a distinct risk of such a side effect being caused by terfenadine itself.

### Example 4

**Oral Formulation - Capsules:**

| Formula | Quantity per capsule in mg. | | |
|---|---|---|---|
| | A | B | C |
| Active ingredient (S) Terfenadine carboxylate | 30.0 | 60.0 | 90.0 |
| Starch 1500 | 69.0 | 39.0 | 9.0 |
| Magnesium Stearate BP | 1.0 | 1.0 | 1.0 |
| Compression Weight | 100.0 | 100.0 | 100.0 |

The active ingredient, which can instead be (R)terfenadine carboxylate or racemic terfenadine carboxylate, is sieved and blended with the excipients. The mixture is filled into suitably sized two-piece hard gelatin capsules using suitable machinery. Other doses may be prepared by altering the fill weight and if necessary, changing the capsule size to suit.

### Example 5

**Oral Formulation - Tablets:**

| Formula | Quantity per Tablet in mg. | | |
|---|---|---|---|
| | A | B | C |
| Active ingredient, (R) Terfenadine Carboxylate | 30.0 | 60.0 | 90.0 |
| Lactose BP | 123.5 | 93.5 | 63.5 |
| Starch BP | 30.0 | 30.0 | 30.0 |
| Pregelatinized Maize Starch BP | 15.0 | 15.0 | 15.0 |
| Magnesium stearate | 1.5 | 1.5 | 1.5 |
| Compression Weight | 200.0 | 200.0 | 200.0 |

The active ingredient, which can instead be (S)terfenadine carboxylate or racemic terfenadine carboxylate, is sieved through a suitable sieve and blended with the lactose until a uniform blend is formed. Suitable volumes of water are added and the powders are granulated. After drying, the granules are then screened and blended with the magnesium stearate. The resulting granules are then compressed into tablets of desired shape. Tablets of other strengths may be prepared by altering the ratio of active ingredient to the excipient(s) or the compression weight.

## Claims

1. Use of a composition comprising a compound of formula I: 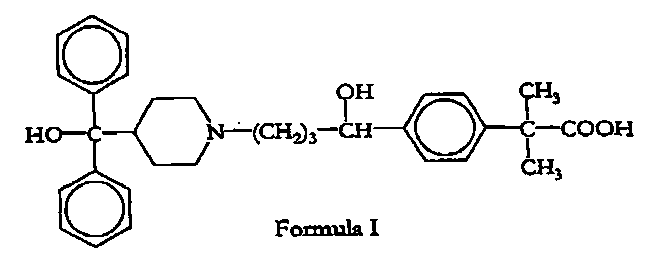 or a pharmaceutically acceptable salt thereof, for the preparation of a medicament for use in providing symptomatic relief from dermal irritation associated with an allergic disorder, cough, cold or flu, wherein the induction of cardiac arrhythmia is avoided, said treatment comprising administering a therapeutically effective amount of a compound of formula I to a human patient whose hepatic function is not impaired.

2. The use of claim 1, wherein the treatment comprises administration of a compound of formula I in an amount of 1 to 500 mg/day.

3. The use of claim 1 or 2, wherein the treatment comprises administration of a compound of formula I in an amount of 20 to 200 mg/day.

4. The use of claim 1, 2 or 3, wherein the treatment comprises the administration of a compound of formula I orally, in single or divided doses.

5. The use of any one of the preceding claims, wherein the compound of formula I is in the form of a single optical isomer and the composition is substantially free of the other such isomer.

6. The use of claim 5, wherein the compound of formula I comprises 90% or more by weight of the R-(+) stereoisomer.

7. The use of claim 5, wherein the compound of formula I comprises 90% or more by weight of the S-(-) stereoisomer.

8. The use of any one of the proceeding claims, wherein the compound of formula I is terfenadine carboxylate.

9. The use of any one of claims 1 to 7, wherein the compound of formula I is a pharmaceutically acceptable salt of terfenadine carboxylate and an inorganic acid selected from the group consisting of hydrochloric acid, hydrobromic acid, hydroiodic acid, sulphuric acid and phosphoric acid.

10. The use of any one of the preceding claims, wherein the composition further comprises a pharmaceutically acceptable carrier or excipient.

11. The use of any one of the preceding claims, wherein the dermal irritation is associated with an allergic disorder.

12. The use of any one of claims 1 to 10, wherein the dermal irritation is associated with solar urticaria or symptomatic dermographism.

13. The use of any one of the preceding claims, wherein said symptomatic relief is associated with the composition's activity as an antihistamine.

14. The use of any one of the preceding claims, wherein the composition further comprises a therapeutically effective amount of a non-steroidal anti-inflammatory agent or a non-narcotic analgesic.

15. The use of claim 14, wherein the composition comprises from 20 mg to 200 mg of a compound of formula I and from 25 mg to 600 mg of the anti-inflammatory agent or analgesic.

16. A pharmaceutical composition in the form of an oral solid preparation comprising 20 to 200 mg of a pharmaceutically acceptable salt of a compound of formula I: 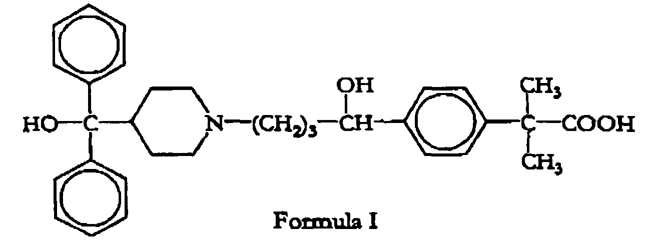 and an inorganic acid selected from the group consisting of hydrochloric acid, hydrobromic acid, hydroiodic acid, sulphuric acid and phosphoric acid, and a pharmaceutically acceptable carrier or excipient, for use in treating dermal irritation, wherein the induction of cardiac arrhythmia is avoided.

17. A pharmaceutical composition as claimed in claim 16, in the form of a tablet, cachet or capsule providing a unit dosage of 30 mg, 60 mg or 90 mg of the pharmaceutically acceptable salt of the compound of formula I.

18. A pharmaceutical composition as claimed in claim 16 or 17, wherein the compound of formula I is in the form of a single optical isomer and the composition is substantially free of the other such isomer.

19. A pharmaceutical composition as claimed in claim 18, wherein the compound of formula I comprises 90% or more by weight of the R-(+) stereoisomer.

20. A pharmaceutical composition as claimed in claim 18, wherein the compound of formula I comprises 90% or more by weight of the S-(-) stereoisomer.

21. A pharmaceutical composition as claimed in any one of the preceding claims, further comprising a therapeutically effective amount of a non-steroidal anti-inflammatory agent or a non-narcotic analgesic.

22. A pharmaceutical composition as claimed in claim 21, comprising from 25 mg to 600 mg of the anti-inflammatory agent or analgesic.

23. A pharmaceutical composition in the form of an oral solid preparation comprising 20 to 200 mg of a pharmaceutically acceptable salt of a compound of formula I: 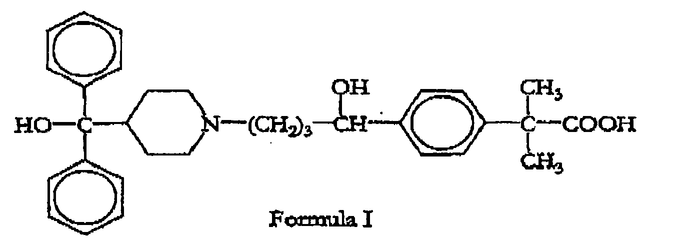 and an inorganic acid selected from the group consisting of hydrochloric acid, hydrobromic acid, hydroiodic acid, sulphuric acid and phosphoric acid, and a pharmaceutically acceptable carrier or excipient.

24. Use of a pharmaceutically acceptable salt of a compound of formula I: 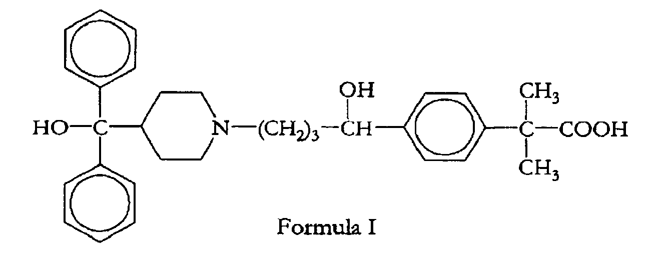 and an inorganic acid selected from the group consisting of hydrochloric acid, hydrobromic acid, hydroiodic acid, sulphuric acid and phosphoric acid, for the preparation of a pharmaceutical composition for the treatment of dermal irritation wherein the induction of cardiac arrhythmia is avoided, wherein the pharmaceutical composition is in the form of an oral solid preparation comprising 20 to 200 mg of the pharmaceutically acceptable salt of the compound of formula I and a pharmaceutically acceptable carrier or excipient.

## Patentansprüche

1. Verwendung einer Zusammensetzung umfassend eine Verbindung der Formel I: 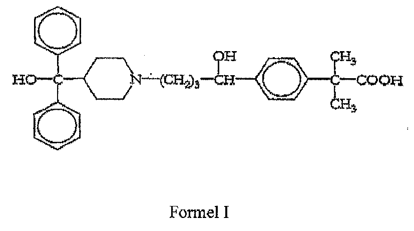 oder eines pharmazeutisch akzeptablen Salzes derselben für die Herstellung eines Medikaments zur Verwendung zum Bieten symptomatischer Erleichterung bei Hautreizung, die mit einer allergischen Störung verbunden ist, Husten, Erkältung oder Grippe, wobei die Auslösung einer Herzrhythmusstörung vermieden wird, wobei die Behandlung das Verabreichen einer therapeutisch wirksamen Menge einer Verbindung der Formel I einem menschlichen Patienten umfasst, dessen Leberfunktion nicht beeinträchtigt ist.

2. Verwendung nach Anspruch 1, wobei die Behandlung die Verabreichung einer Verbindung der Formel I in einer Menge von 1 bis 500 mg/Tag umfasst.

3. Verwendung nach Anspruch 1 oder 2, wobei die Behandlung die Verabreichung einer Verbindung der Formel I in einer Menge von 20 bis 200 mg/Tag umfasst.

4. Verwendung nach Anspruch 1, 2 oder 3, wobei die Behandlung die Verabreichung einer Verbindung der Formel I oral in einer einzigen oder in fraktionierten Dosen umfasst.

5. Verwendung nach einem der vorhergehenden Ansprüche, wobei die Verbindung der Formel I in Form eines einzigen optischen Isomers vorliegt und die Zusammensetzung im Wesentlichen vom anderen derartigen Isomer frei ist.

6. Verwendung nach Anspruch 5, wobei die Verbindung der Formel I 90 Gew.-% oder mehr des R-(+)-Stereoisomers umfasst.

7. Verwendung nach Anspruch 5, wobei die Verbindung der Formel I 90 Gew.-% oder mehr des S-(-)-Stereoisomers umfasst.

8. Verwendung nach einem der vorhergehenden Ansprüche, wobei die Verbindung der Formel I Terfenadincarboxylat ist.

9. Verwendung nach einem der Ansprüche 1 bis 7, wobei die Verbindung der Formel I ein pharmazeutisch akzeptables Salz von Terfenadincarboxylat und einer anorganischen Säure ausgewählt aus der Gruppe bestehend aus Salzsäure, Bromwasserstoffsäure, Iodwasserstoffsäure, Schwefelsäure und Phosphorsäure ist.

10. Verwendung nach einem der vorhergehenden Ansprüche, wobei die Zusammensetzung des Weiteren einen pharmazeutisch akzeptablen Träger oder ein pharmazeutisch akzeptables Vehikel umfasst.

11. Verwendung nach einem der vorhergehenden Ansprüche, wobei die Hautreizung mit einer allergischen Störung verbunden ist.

12. Verwendung nach einem der Ansprüche 1 bis 10, wobei die Hautreizung mit Sonnenurtikaria oder symptomatischem Dermographismus verbunden ist.

13. Verwendung nach einem der vorhergehenden Ansprüche, wobei die symptomatische Erleichterung mit der Aktivität der Zusammensetzung als Antihistamin verbunden ist.

14. Verwendung nach einem der vorhergehenden Ansprüche, wobei die Zusammensetzung des Weiteren eine therapeutisch wirksame Menge eines nichtsteroidalen antiinflammatorisch wirkenden Mittels oder eines nicht narkotisch wirkenden schmerzstillenden Mittels umfasst.

15. Verwendung nach Anspruch 14, wobei die Zusammensetzung 20 mg bis 200 mg einer Verbindung der Formel I und 25 mg bis 600 mg des antiinflammatorisch wirkenden Mittels oder schmerzstillenden Mittels umfasst.

16. Pharmazeutische Zusammensetzung in Form einer oralen festen Zusammensetzung umfassend 20 bis 200 mg eines pharmazeutisch akzeptablen Salzes einer Verbindung der Formel I: 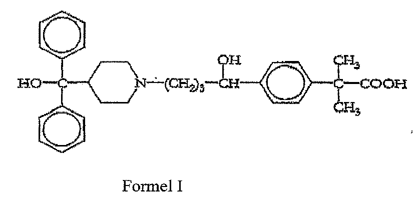 und einer anorganischen Säure ausgewählt aus der Gruppe bestehend aus Salzsäure, Bromwasserstoffsäure, Iodwasserstoffsäure, Schwefelsäure und Phosphorsäure und einen pharmazeutisch akzeptablen Träger oder ein pharmazeutisch akzeptables Vehikel zur Verwendung bei der Behandlung von Hautreizung, wobei die Auslösung einer Herzrhythmusstörung vermieden wird.

17. Pharmazeutische Zusammensetzung nach Anspruch 16 in Form einer Tablette, Obladenkapsel oder Kapsel, die eine Einheitsdose von 30 mg, 60 mg oder 90 mg des pharmazeutisch akzeptablen Salzes der Verbindung der Formel I bereitstellt.

18. Pharmazeutische Zusammensetzung nach Anspruch 16 oder 17, wobei die Verbindung der Formel I in Form eines einzigen optischen Isomers vorliegt und die Zusammensetzung im Wesentlichen von dem anderen derartigen Isomer frei ist.

19. Pharmazeutische Zusammensetzung nach Anspruch 18, wobei die Verbindung der Formel I 90 Gew.- % oder mehr des R-(+)-Stereomers umfasst.

20. Pharmazeutische Zusammensetzung nach Anspruch 18, wobei die Verbindung der Formel I 90 Gew.- % oder mehr des S-(-)-Stereomers umfasst.

21. Pharmazeutische Zusammensetzung nach einem der vorhergehenden Ansprüche, des Weiteren eine therapeutisch wirksame Menge eines nichtsteroidalen antiinflammatorisch wirkenden Mittels oder eines nicht narkotisch wirkenden schmerzstillenden Mittels umfassend.

22. Pharmazeutische Zusammensetzung nach Anspruch 21, umfassend 25 mg bis 600 mg des antiinflammatorisch wirkenden Mittels oder schmerzstillenden Mittels.

23. Pharmazeutische Zusammensetzung in Form einer oralen festen Zusammensetzung umfassend 20 bis 200 mg eines pharmazeutisch akzeptablen Salzes einer Verbindung der Formel I: 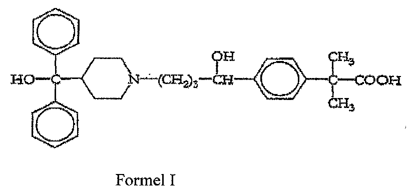 und einer anorganischen Säure ausgewählt aus der Gruppe bestehend aus Salzsäure, Bromwasserstoffsäure, Iodwasserstoffsäure, Schwefelsäure und Phosphorsäure und einen pharmazeutisch akzeptablen Träger oder ein pharmazeutisch akzeptables Vehikel.

24. Verwendung eines pharmazeutisch akzeptablen Salzes einer Verbindung der Formel I 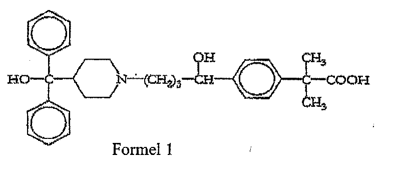 und einer anorganischen Säure ausgewählt aus der Gruppe bestehend aus Salzsäure, Bromwasserstoffsäure, Iodwasserstoffsäure, Schwefelsäure und Phosphorsäure für die Herstellung einer pharmazeutischen Zusammensetzung für die Behandlung von Hautreizung, wobei die Auslösung einer Herzryhthmusstörung vermieden wird, wobei die pharmazeutische Zusammensetzung in Form einer oralen festen Zubereitung umfassend 20 bis 200 mg des pharmazeutisch akzeptablen Salzes der Verbindung der Formel I und eines pharmazeutisch akzeptablen Trägers oder Vehikels vorliegt.

## Revendications

1. Utilisation d'une composition comprenant un composé de formule I : 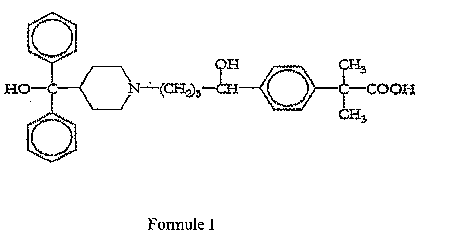 ou un sel de ce dernier pharmaceutiquement acceptable, pour la préparation d'un médicament à utiliser pour le soulagement symptomatique de l'irritation dermique associée à un trouble allergique, à une toux, à un rhume ou à la grippe, dans laquelle l'induction d'une arythmie cardiaque est évitée, ledit traitement comprenant l'administration d'une quantité thérapeutiquement efficace d'un composé de formule I à un patient humain dont la fonction hépatique n'est pas altérée.

2. Utilisation de la revendication 1, dans laquelle le traitement comprend l'administration d'un composé de formule I en une quantité de 1 à 500 mg/jour.

3. Utilisation de la revendication 1 ou 2, dans laquelle le traitement comprend l'administration d'un composé de formule I en une quantité de 20 à 200 mg/jour.

4. Utilisation de la revendication 1, 2 ou 3, dans laquelle le traitement comprend l'administration d'un composé de formule I par voie orale, en une dose unique ou en doses fractionnées.

5. Utilisation de l'une quelconque des revendications précédentes, dans laquelle le composé de formule I se présente sous la forme d'un simple isomère optique et la composition est substantiellement exempte de l'autre tel isomère.

6. Utilisation de la revendication 5, dans laquelle le composé de formule 1 comprend 90 % ou plus, en poids, du stéréoisomère R-(+).

7. Utilisation de la revendication 5, dans laquelle le composé de formule I comprend 90 % ou plus, en poids, du stéréoisomère S-(-).

8. Utilisation selon l'une quelconque des revendications précédentes, dans laquelle le composé de formule I est le carboxylate de terfénadine.

9. Utilisation selon l'une quelconque des revendications 1 à 7, dans laquelle le composé de formule I est un sel du carboxylate de terfénadine pharmaceutiquement acceptable et un acide inorganique sélectionné parmi le groupe constitué par l'acide chlorhydrique, l'acide hydrobromique, l'acide hydroïodique, l'acide sulfurique et l'acide phosphorique.

10. Utilisation de l'une quelconque des revendications précédentes, dans laquelle la composition contient, en outre, un vecteur ou excipient pharmaceutiquement acceptable.

11. Utilisation de l'une quelconque des revendications précédentes, dans laquelle l'irritation dermique est associée à un trouble allergique.

12. Utilisation de l'une quelconque des revendications 1 à 10, dans laquelle l'irritation dermique est associée à l'urticaire solaire ou à un dermographisme symptomatique.

13. Utilisation de l'une quelconque des revendications précédentes, dans laquelle le soulagement symptomatique est associé à l'activité antihistaminique de la composition.

14. Utilisation de l'une quelconque des revendications précédentes, dans laquelle la composition comprend, en outre, une quantité d'un agent anti-inflammatoire non stéroïdien ou d'un analgésique non narcotique thérapeutiquement efficace.

15. Utilisation de la revendication 14, dans laquelle la composition comprend 20 mg à 200 mg d'un composé de formule 1 et 25 mg à 600 mg de l'agent anti-inflammatoire ou de l'analgésique.

16. Composition pharmaceutique sous la forme d'une préparation solide orale comprenant 20 à 200 mg d'un sel pharmaceutiquement acceptable d'un composé de formule I : 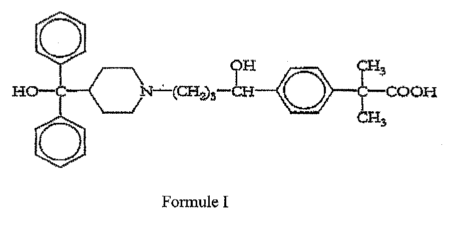 et un acide inorganique sélectionné parmi le groupe constitué par l'acide chlorhydrique, l'acide hydrobromique, l'acide hydroïodique, l'acide sulfurique et l'acide phosphorique, et un vecteur ou excipient pharmaceutiquement acceptable, à utiliser pour le traitement de l'irritation dermique, dans lequel l'induction d'une arythmie cardiaque est évitée.

17. Composition pharmaceutique telle que revendiquée dans la revendication 16, sous la forme d'un comprimé, d'un cachet ou d'une gélule, apportant une dose unitaire de 30 mg, 60 mg ou 90 mg du sel pharmaceutiquement acceptable du composé de formule I.

18. Composition pharmaceutique telle que revendiquée dans la revendication 16 ou 17, dans laquelle le composé de formule I est sous la forme d'un simple isomère optique et la composition est substantiellement exempte de l'autre tel isomère.

19. Composition pharmaceutique telle que revendiquée dans la revendication 18, dans laquelle le composé de formule I comprend 90 % ou plus, en poids, du stéréoisomère R-(+).

20. Composition pharmaceutique telle que revendiquée dans la revendication 18, dans laquelle le composé de formule I contient 90 % ou plus, en poids, du stéréoisomère S-(-).

21. Composition pharmaceutique telle que revendiquée dans l'une quelconque des revendications précédentes contenant, en outre, une quantité d'un agent anti-inflammatoire non stéroïdien ou d'un analgésique non narcotique thérapeutiquement efficace.

22. Composition pharmaceutique telle que revendiquée dans la revendication 21, comprenant 25 mg à 600 mg de l'agent anti-inflammatoire ou de l'analgésique.

23. Composition pharmaceutique sous la forme d'une préparation solide orale comprenant 20 à 200 mg d'un sel pharmaceutiquement acceptable d'un composé de formule I : 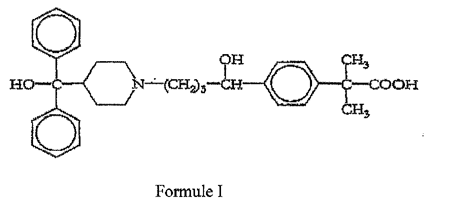 et un acide inorganique sélectionné parmi le groupe constitué par l'acide chlorhydrique, l'acide hydrobromique, l'acide hydroïodique, l'acide sulfurique et l'acide phosphorique, et un vecteur ou excipient pharmaceutiquement acceptable.

24. Utilisation d'un sel pharmaceutiquement acceptable d'un composé de formule 1 : 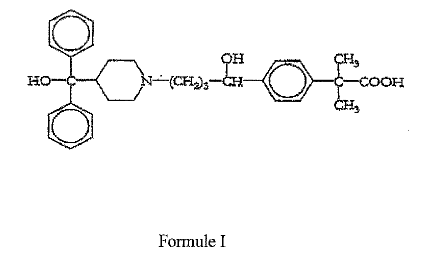 et un acide inorganique sélectionné parmi le groupe constitué par l'acide chlorhydrique, l'acide hydrobromique, l'acide hydroïodique, l'acide sulfurique et l'acide phosphorique pour la préparation d'une composition pharmaceutique pour le traitement de l'irritation dermique dans lequel l'induction de l'arythmie cardiaque est évité, dans laquelle la composition pharmaceutique est sous la forme d'une préparation solide orale comprenant 20 à 200 mg du sel pharmaceutiquement acceptable du composé de formule I et un vecteur ou excipient pharmaceutiquement acceptable.
